# EUROPEAN PATENT SPECIFICATION

(11) **EP 3 138 569 B1**
(45) Date of publication and mention of the grant of the patent: **02.09.2020**
(21) Application number: 15785845.7
(22) Date of filing: 26.01.2015
(51) Int. Cl.: A61K 8/60, A61Q 19/00, A61K 31/7016, A61P 17/00, A61P 17/08

(54) **COMPOSITION FOR EXTERNAL APPLICATION**
ZUSAMMENSETZUNG ZUR ÄUSSEREN ANWENDUNG
COMPOSITION D'APPLICATION EXTERNE

(30) Priority: 28.04.2014 JP 2014092863
(43) Date of publication of application: 08.03.2017
(73) Proprietor: Mitsui Sugar Co., Ltd., Tokyo 13-8423 (JP)
(72) Inventor: MIZU, Masami, Tokyo 103-0015 (JP)
(74) Representative: Grünecker Patent- und Rechtsanwälte PartG mbB
(86) International application number: PCT/JP2015/052060
(87) International publication number: WO 2015/166677

(56) References cited:
- WO-A1-2010/004553
- JP-A- 2006 143 605
- JP-A- 2009 079 045
- JP-A- 2010 111 613
- JP-A- 2010 528 595
- JP-A- 2011 522 548
- US-A1- 2013 108 565
- US-A1- 2013 287 714
- US-B2- 7 413 755
- TAKEDA E1 ET AL: "Control of oxidative stress and metabolic homeostasis by the suppression of postprandial hyperglycemia", TOKUSHIMA JOURNAL OF EXPERIMENTAL MEDICINE, TOKUSHIMA DAIGAKU IGAKUBU, TOKUSHIMA, JP , vol. 52, no. Suppl 1 November 2005 (2005-11-01), pages 259-265, XP009517250, ISSN: 0040-8875, DOI: 10.2152/JMI.52.259 Retrieved from the Internet: URL:https://pdfs.semanticscholar.org/7484/ c45e326067aa9b8d40f25b5a0fcb301dca89.pdf?_ ga=2.170684329.844528113.1573802875-928644 479.1573802875

## Description

### Technical Field

The present invention relates to a cosmetic use of a composition for external application.

### Background Art

It is known that fibroblasts are one type of cells which constitute connective tissues and produce the components which constitute epidermis such as collagen, elastin, and hyaluronic acid. In recent years, the effect of anti-aging through the reconstitution of the epidermis by collagen, elastin, hyaluronic acid, and others have been expected; and technological developments, including the growth stimulation of the fibroblasts and the transplantation of the fibroblasts (regenerative medicine) have been advanced.

Specifically, Patent Literature 1 discloses a composition comprising cryptoxanthin and/or an ester thereof and having fibroblast growth-promoting effect and/or collagen production-promoting effect. Further, Patent Literature 2 discloses an external preparation for skin comprising cryptoxanthin and/or a derivative thereof.

Patent Literature 3 provides an improved antioxidant based on trehalulose and optionally isomaltulose for foods, feeds, cosmetics and pharmaceuticals and compositions containing this antioxidant which may minimize the harmful process of oxidation and increase storage stability.

Patent Literature 4 discloses the use of a cotton honeydew extract comprising trehalulose. The compositions described in Patent Literature 4 are intended to reinforce keratin of the hair and skin and to protect against the effects of light.

Patent Literature 5 relates to a transdermal agent which promotes the proliferation of a fibroblast. The transdermal agent preferably includes an isomalto-oligosaccharide.

### Citation List

### Patent Literature

Patent Literature 1: Japanese Unexamined Patent Publication No.2008-297216
Patent Literature 2: Japanese Unexamined Patent Publication No.2010-254591
Patent Literature 3: Japanese Unexamined Patent Publication No.2011-522548
Patent Literature 4: US-B-7,413,755
Patent Literature 5: Japanese Unexamined Patent Publication No.2006-143605

### Summary of Invention

### Problems to be Solved by the Invention

Although certain compositions and external skin preparations are known that promote the growth of fibroblasts, it cannot be said that there have been ample selections to satisfy the diverse needs of the consumers so far.

Therefore, the present invention has an object of providing a novel composition for external application capable of activating the fibroblasts.

### Means for Solving the Problems

The above object is solved by appended claims 1 to 4. The present invention further describes a composition for external application comprising at least one component selected from the group consisting of trehalulose, isomaltulose, and reduced isomaltulose.

Since the composition for external application comprises trehalulose, isomaltulose, or reduced isomaltulose, it can activate fibroblasts. The fibroblasts are one type of cells which constitute connective tissues and produce the components which constitute epidermis such as collagen, elastin, and hyaluronic acid. Consequently, the composition for external application can promote the repair and regeneration (or reconstitution) of the epidermis through the activation of the fibroblasts, and the improving effect of wrinkles and sagging, the anti-aging effect and others can be expected.

Further, since the composition for external application comprises trehalulose, isomaltulose, or reduced isomaltulose, it can also reduce oxidative stress induced cell damage. Consequently, according to the composition for external application, the effect of reducing cancer risk resulting from the cell damage as well as the effect of preventing the aging of the skin and others can be expected.

Furthermore, since the composition for external application comprises trehalulose, isomaltulose, or reduced isomaltulose, it can inhibit the activity of tyrosinase. Tyrosinase is expressed in melanocytes and is an enzyme that catalyzes the synthetic reaction of melanin. Consequently, the composition for external application prevents the excessive accumulation of melanin, and the effect of preventing the formation of skin spots and freckles as well as skin-whitening effect and others can be expected.

Since the composition for external application exerts the respective effects mentioned above, it is a cosmetic and more preferably is for dermal use.

In addition, since the composition for external application exerts the respective effects mentioned above, it is preferably used in the utilities of activating the fibroblasts, reducing the oxidative stress induced cell damage, or inhibiting tyrosinase.

Specifically, the present invention also describes an agent for activating fibroblast comprising at least one component selected from the group consisting of trehalulose, isomaltulose, and reduced isomaltulose.

The present invention further describes an agent for reducing the oxidative stress induced cell damage comprising at least one component selected front the group consisting of trehalulose, isomaltulose, and reduced isomaltulose.

The aforementioned stress may be oxidative stress by ultraviolet rays.

The present invention furthermore describes an agent for inhibiting tyrosinase comprising at least one component selected from the group consisting of trehalulose, isomaltulose, and reduced isomaltulose.

According to the present invention the composition is for use in a method of activating the fibroblasts, a method of reducing the oxidative stress induced cell damage, a method of inhibiting tyrosinase, a method of improving wrinkles or sagging, a method of preventing the formation of skin spots or freckles, a method of preventing aging of the skin resulting from the cell damage, or a method of skin whitening, each of which involves delivering to at least a part of the skin or the nail, the composition for external application comprising as an effective ingredient, at least one component selected from the group consisting of trehalulose, isomaltulose, and reduced isomaltulose.

Moreover, described herein is the use of at least one component selected from the group consisting of trehalulose, isomaltulose, and reduced isomaltulose in the manufacture of an agent for activating fibroblast, an agent for reducing the oxidative stress induced cell damage, an agent for inhibiting tyrosinase, an agent for improving wrinkles or sagging, an agent for preventing the formation of skin spots or freckles, an agent for preventing aging of the skin resulting from the cell damage, or an agent for whitening skin, each of which consists of the composition for external application.

### Effects of the Invention

The present invention describes a novel composition for external application capable of activating fibroblasts. According to the composition for external application, the repair and regeneration (or reconstitution) of epidermis can be promoted through the activation of fibroblasts, and the effect of improving wrinkles and sagging and others can be expected. Therefore, it is suitable for anti-aging.

The composition for external application can also reduce the oxidative stress induced cell damage in addition to the fibroblast-activating effect. Ultraviolet rays (especially, UVB) are known to generate oxidative stress substances such as reactive oxygen species in cells. Such oxidative stress can be a cause for carcinogenesis or aging through the cell damage such as cell death. It can be expected that the composition for external application according the present invention prevents aging of the skin resulting from the cell damage.

The composition for external application can further inhibit tyrosinase in addition to the fibroblast-activating effect. It is known that melanocytes, which are the cells that form melanin, have tyrosinase and produce melanin from tyrosine. It is known that the melanin has the function of preventing the damage of somatic cells by ultraviolet rays; however, its excessive accumulation is a cause for pigmentation such as skin spots. The composition for external application prevents the excessive accumulation of melanin through the tyrosinase inhibition, and the prevention of the formation of skin spots and freckles and the skin-whitening effect can be expected.

### Brief Description of Drawings

FIG. 1 is a graph showing the evaluation results of the fibroblast-activating effect.
FIG.2 is a graph showing the evaluation results of the reducing effect of the oxidative stress induced cell damage.
FIG. 3 is a graph showing the evaluation results of the tyrosinase-inhibiting effect.
FIG. 4 is a graph showing the evaluation results of the reducing effect of the oxidative stress induced cell damage.

### Embodiments for Carrying Out the Invention

Embodiments for carrying out the present invention will be described below in details.

The composition for external application comprises at least one component selected from the group consisting of trehalulose, isomaltulose, and reduced isomaltulose.

Trehalulose is a compound which is also referred to as 1-O-α-D-glucopyranosyl-D-fructose. Trehalulose is naturally found in beeswax. It is also present in transglycosylated products that are generated when α-glucosyltransferase (isomaltulose synthase) derived from a bacterium or yeast acts on sucrose.

As trehalulose, naturally-derived ones may be used, or ones that are synthesized by enzymatic actions may be used. Alternatively, commercial ones may be used as trehalulose. Examples of the commercial products include trehalulose syrups (trade name - "MILDIA-75" and "MILDIA-85" manufactured by Mitsui Sugar Co., Ltd.).

When the composition for external application comprises trehalulose, the content of trehalulose may be from 0.3 to 20 w/v%, preferably from 0.3 to 10 w/v%, more preferably from 0.3 to 5 w/v%, further preferably from 1.25 to 5 w/v%, and further more preferably from 1.5 to 5 w/v%, relative to the total volume of the composition for external application. If the content of trehalulose is 1.25 w/v% or more, the effects by the present invention will be exerted more markedly. In addition, if the content of trehalulose is 20 w/v% or less, the cytotoxicity will be more reduced.

Isomaltulose is a compound which is also referred to as 6-O-α-D-glucopyranosyl-D-fructose. In addition, isomaltulose is further referred to as palatinose. Furthermore, "PALATINOSE" is a registered trademark of Mitsui Sugar Co., Ltd.

Isomaltulose is naturally found in beeswax. It is also present in transglucosylation products that are generated when α-glucosyltransferase (isomaltulose synthase) derived from a bacterium or yeast acts on sucrose. On an industrial scale, palatinose is manufactured by allowing an α-glucosyltransferase derived from a bacterium such as *Protaminobacter rubrum* or *Serratia plymuthica* to act on sucrose.

As isomaltulose, naturally-derived ones may be used, or ones that are synthesized by enzymatic actions may be used. Alternatively, commercial ones may be used as isomaltulose. Examples of the commercial products include crystalline palatinose (trade name - "Crystalline Palatinose IC" manufactured by Mitsui Sugar Co., Ltd.), crystalline palatinose (trade name - "Crystalline Palatinose PST-N" manufactured by BENEO GmbH), powder palatinose (trade name - "Powder Palatinose ICP" manufactured by Mitsui Sugar Co., Ltd.), palatinose syrup (trade name - "Palatinose Syrup-ISN" and "Palatinose Syrup-TN" manufactured by Mitsui Sugar Co., Ltd.).

When the composition for external application comprises isomaltulose, the content of isomaltulose may be from 0.04 to 20 w/v%, preferably from 0.09 to 20 w/v%, more preferably from 0.19 to 20 w/v%, further preferably from 0.6 to 20 w/v%, and further more preferably from 0.75 to 5 w/v%, relative to the total volume of the composition for external application. If the content of isomaltulose is 0.75 w/v% or more, the effects of the present invention will be exerted more markedly. In addition, if the content of isomaltulose is 20 w/v% or less, the cytotoxicity will be more reduced. Moreover, when the composition for external application is at least intended for exerting the reducing effect cell damage, the content of isomaltulose may be less: for example, it is preferably from 0.04 to 10 w/v%, more preferably from 0.04 to 5 w/v%, and further preferably from 0.04 to 2 w/v%.

Reduced isomaltulose is a reduced form obtained by subjecting isomaltulose to hydrogenation. The reduced isomaltulose is also referred to as "REDUCED PALATINOSE" (registered trademark of Mitsui Sugar Co., Ltd.). The reduced isomaltulose is principally a mixture of α-D-glycopyranosyl-1,1-mannitol (which will be also referred to as "GPM" hereafter) and α-D-glycopyranosyl-1,6-sorbitol (which will be also referred to as "GPS" hereafter).

The mixing ratio of GPM and GPS in the reduced isomaltulose is not particularly limited and may be from 1:99 to 99:1,. It may also be a mixture of nearly equimoles of GPM and GPS (from 4:6 to 6:4 and 95% or more based on the weight ratio of dried solid portions); it may even be a mixture that is rich in either of GPM and GPS.

There is a difference in the solubility toward water between GPM and GPS. The above-described mixing ratio can be adjusted by taking advantage of the difference in solubility between the two components (see, for example, Japanese Unexamined Patent Publication No. H10-310595). Specifically, since the solution equilibrium is temperature-dependent, the concentration ratio of the two components can be adjusted by controlling the temperature of an aqueous solution of the reduced isomaltulose. After adjustment to the solution equilibrium, the rate of temperature variation can be fixed to control the concentrations of GPM and GPS in the reduced isomaltulose.

Reduced isomaltulose may be in a granular form. The granular reduced isomaltulose can be, for example, produced in the manner that follows. A solution of reduced isomaltulose is first heated at reduced pressure to obtain a concentrated liquid. This concentrated liquid is next sprayed to the inner wall of a slowly rotating drum at reduced pressure. The reduced isomaltulose adhered to the inner wall surface is scraped off with an agitator. The reduced isomaltulose that has been scraped off is further granulated to granules by the rotation of the drum, whereby the granular form can be made.

As reduced isomaltulose, commercial ones can be used. Examples of the commercial products include Palatinit PN (Mitsui Sugar Co., Ltd.), Powdered Palatinit PNP (Mitsui Sugar Co., Ltd.), Palatinit GS(Mitsui Sugar Co., Ltd.), Powdered Palatinit GSP (Mitsui Sugar Co., Ltd.), and Palatinit ST-F (Mitsui Sugar Co., Ltd.).

When the composition for external application comprises reduced isomaltulose, the content of the reduced isomaltulose may be from 0.125 to 20 w/v%, preferably from 0.125 to 5 w/v%, and more preferably from 0.3 to 5 w/v%, relative to the total volume of the composition for external application. If the content of reduced isomaltulose is 0.3 w/v% or more, the effects of the present invention will be exerted more markedly. In addition, if the content of reduced isomaltulose is 20 w/v% or less, the cytotoxicity will be more reduced.

The composition for external application according to the present embodiment may comprise at least one component of the above-described ingredients or may comprise two or more components in combination.

The composition for external application according to the present embodiment may further comprise ingredients having the fibroblast-activating effect, the reducing effect of the oxidative stress induced cell damage, or the tyrosinase-inhibiting effect, in addition to trehalulose, isomaltulose, and reduced isomaltulose. Examples of the ingredient having the fibroblast-activating effect include the loquat extract, which is disclosed in Japanese Examined Patent Publication No. H5-17206, α-hydroxyacetic acid, a sterol ester of α-hydroxyacid, 6-benzylaminopurine, certain ribonucleases, which are disclosed in Japanese Unexamined Patent Publication No. H7-309778, L-lysyl-L-glycyl-L-histidine, the fibroblast growth factor derived from mammalian milk, which is disclosed in Japanese Unexamined Patent Publication No. H8-119867, and the coemzyme A of oxidative type, which is disclosed in Japanese Unexamined Patent Publication No. H8-175961. Examples of the ingredient having the reducing effect of the oxidative stress induced cell damage include antioxidants derived from natural products. For example, there are specifically mentioned: vitamins such as L-ascorbic acid (vitamin C) and tocopherols (vitamin E); polyphenols such as anthocyanin and chlorophill; and flavonoids such as catechin. Examples of the ingredient having the tyrosinase-inhibiting effect include ascorbic acids, hydrogen peroxide, hydroquinone, catechol, sulfurs, kojic acid, arbutin, glutathione, o-coumaric acid-β-D-glycoside, and the extract of roasted chestnut shells, which is disclosed in Japanese Unexamined Patent Publication No. 2007-126406.

The composition for external application according to the present embodiment can be used as a drug, a quasi-drug (e.g., a medicinal cosmetic), or a cosmetic.

The composition for external application according to the present embodiment may further comprise other ingredients, for example, which are normally added to the drug, quasi-drug, or cosmetic, depending on its utility. Examples of the other ingredients include oil-based agents, surfactants, moisturizing components, thickeners, powders, inorganic pigments, organic dyes, organic powders, ultraviolet absorbers, lower alcohols, vitamins, proteins, and lipids.

Examples of the oil-based agent include oils, hydrocarbons, higher fatty acids, higher alcohols, synthetic ester oils, and silicone oils. Additionally, examples of the oil include macadamia nut oil, avocado oil, corn oil, olive oil, rapeseed oil, sesame oil, castor oil, safflower oil, cotton seed oil, jojoba oil, coconut oil, palm oil, liquid lanolin, hydrogenated coconut oil, hydrogenated oil, Japanese wax, hydrogenated castor oil, bees wax, candelilla wax, carnauba wax, privet wax, lanolin, reduced lanolin, hardened lanolin, and jojoba wax. Examples of the hydrocarbon include waxes, liquid paraffin, squalane, pristane, ozokerite, paraffin, ceresin, vaseline, and microcrystalline wax. Examples of the higher fatty acid include oleic acid, isostearic acid, lauric acid, myristic acid, palmitic acid, stearic acid, behenic acid, and undecylenic acid. Examples of the higher alcohol include cetyl alcohol, stearyl alcohol, isostearyl alcohol, behenyl alcohol, octyldodecanol, myristyl alcohol, and cetostearyl alcohol. Examples of the synthetic ester include cetyl isooctanate, isopropyl myristate, hexyldodecyl isostearate, diisopropyl adipate, di-2-ethylhexyl sebacate, cetyl lactate, diisostearyl malate, ethylene glycol di-2-ethylhexanate, neopentyl glycol dicaprate, glycerol di-2-hepthyl undecanate, glycerol tri-2-ethyl hexanate, trimethylolpropane tri-2-ethyl hexanate, trimethylolpropane triisostearate, and pentaerythrit tetra-2-ethylhexanate. Examples of the silicone oil include chain polysiloxanes such as dimethylpolysiloxane, methylphenylpolysiloxane, and diphenylpolysiloxane; cyclic polysiloxanes such as octamethylcyclotetrasiloxane, decamethylcyclopentasiloxane, and dodecamethylcycloxasiloxane; and modified polysiloxanes such as amino-modified polysiloxane, polyether-modified polysiloxane, alkyl-modified polysiloxane, and fluoro-modified polysiloxane.

Examples of the surfactant include anionic surfactants, cationic surfactants, amphoteric surfactants, and non-ionic surfactants. Further, examples of the anionic surfactant include fatty acid soap (e.g., sodium laurate and sodium palmitate), potassium laurylsulfate, and alkylsulfate triethanol amine ether. Examples of the cationic surfactant include stearyl trimethyl ammonium chloride, benzalkonium chloride, and laurylamine oxide. Examples of the amphoteric surfactant include imidazoline-based amphoteric surfactants (e.g., 2-cocoyl-2-imidazolium hydroxide-1-carboxyethyloxy 2 sodium salt), betaine-based amphoteric surfactants (e.g., alkylbetaine, amidobetaine, and sulfobetaine), and acylmethyltaurine. Examples of the non-ionic surfactant include sorbitan fatty acid esters (e.g., sorbitan monostearate and sorbitan sesqueoleate), glycerol fatty acids (e.g., glycerol monostearate), propylene glycol fatty acid esters (e.g., propylene glycol monostearate), hydrogenated castor oil derivatives, glycerol alkyl ethers, POE sorbitan fatty acid esters (e.g., POE sorbitan monooleate and polyoxyethylenesorbitan monostearate), POE sorbit fatty acid esters (e.g., POE sorbit monolaurate), POE glycerol fatty acid esters (e.g., POE-glycerol monoisostearate), POE fatty acid esters (e.g., polyethylene glycol monooleate and POE distearate), POE alkyl ethers (e.g., POE 2-octyl dodecyl ether), POE alkyl phenyl ethers (e.g., POE nonyl phenyl ether), pluronic types, POE•POP alkyl ethers (e.g., POE• POP2-decyl tetradecyl ether), TETRONICs, POE castor oil-hydrogenated castor oil derivatives (e.g., POE castor oils, POE hydrogenated castor oils), sucrose fatty acid esters, and alkylglucosides.

Examples of the moisturizing ingredient include: polyhydric alcohols such as polyethylene glycol, glycerol, 1,3-butylene glycol, erythritol, sorbitol, xylitol, maltitol, propylene glycol, dipropylene glycol, diglycerol, isoprene glycol, 1,2-pentanediol, 2,4-hexylene glycol, 1,2-hexanediol, and1,2-octanediol; sodium pyrrolidone carboxylate; lactic acid; and sodium lactate.

Examples of the thickener include guar gum, quince seed, carrageenan, galactan, gum arabic, pectin, mannan, starch, xanthan gum, curdlan, methylcellulose, hydroxyethyl cellulose, carboxymethyl cellulose, methylhydroxypropyl cellulose, chondroitin sulfate, dermatan sulfate, glycogen, heparan sulfate, hyaluronic acid, sodium hyaluronate, gum tragacanth, keratan sulfate, chondroitin, mucoitin sulfate, hydroxyethyl guar gum, carboxymethyl guar gum, dextran, keratosulfate, locust been gum, succinoglucan, charonic acid, chitin, chitosan, carboxymethyl chitin, agar, polyvinyl alcohol, polyvinylpyrrolidone, carboxyvinyl polymer, sodium polyacrylate, polyethylene glycol, and bentonite.

Examples of the powder include mica, talc, kaolin, synthetic mica, calcium carbonate, magnesium carbonate, silic acid anhydride (silica), aluminum oxide, and barium sulfate. These may be surface-treated.

Examples of the inorganic pigment include bengala, yellow iron oxide, black iron oxide, cobalt oxide, ultramarine blue, prussian blue, titanium oxide, and zinc oxide. These may be surface-treated.

Examples of the organic dye include pearl ingredients such as mica titanium, fish scale guanine, and bismuth oxychloride; and Food Red No. 202, Food Red No. 228, Food Red No. 226, Food Yellow No. 4, Food Blue No. 404, Food Yellow No. 5, Food Red No. 505, Food Red No. 230, Food Red No. 223, Food Orange No. 201, Food Red No. 213, Food Yellow No. 204, Food Yellow No. 203, Food Blue No. 1, Food Green No. 201, Food Blue No. 1, Food Green No. 201, Food Purple No. 201, and Food Red No. 204, all of which may be lake dyes. These may be surface-treated.

Examples of the organic powder include polyethylene powder, methyl poly(meth)acrylate, nylon powder, and an organopolysiloxane elastomer.

Examples of the ultraviolet absorber include a para-aminobenzoic acid-based ultraviolet absorber, an anthranilic acid-based ultraviolet absorber, a salicylic acid-based ultraviolet absorber, a benzophenone-based ultraviolet absorber, a sugar-based ultraviolet absorber, 2-(2'-hydroxy-5'-t-octylyphenyl)benzotriazole, and 4-methoxy-4' -t-butylbenzoylmethane.

Examples of the lower alcohol include ethanol and isopropanol.

Examples of the vitamin include vitamin A or a derivative thereof; vitamin Bs such as a vitamin B6 hydrochloride, vitamin B6 tripalmitate, vitamin B6 dioctanoate, vitamin B2 or a derivative thereof, vitamin B12, and vitamin B15 or a derivative thereof; vitamin Es such as α-tocopherol, β-tocopherol, γ-tocopherol, and vitamin E acetate; vitamin Ds; and vitamin H, pantothenic acid, pantethine, and pyrroloquinoline quinone.

Examples of the protein include plant-derived proteins such as wheat protein, soybean protein, and soybean isoflavone; animal-derived proteins such as keratin, a keratin hydrolysate, a sulfone -type keratin, lactoferin, collagen, and elastin; and derivatives of the foregoing as well as salts of the foregoing.

Examples of the lipid include glycerophospholipids such as phosphatidylcholine, phosphatidylethanolamine, phosphatidylserine, phosphatidic acid, phosphatidylglycerol, phosphatidylinositol, cardiolipin, egg yolk lecithin, hydrogenated egg yolk lecithin, soybean lecithin, and hydrogenated soybean lecithin; sphingophospholipids such as sphingomyelin, ceramide phospholylethanolamine, and ceramide phospholylglycerol; plasmalogens; glycerolipids the glycolipid of which is digalactosyldiglyceride and a sulfate of digalactosyldiglyceride; sphingoglycolipids such as glactosylceramide, glactosylceramide sulfate, lactosylceramide, ganglioside G7, ganglioside G6, and ganglioside G4; mixtures of the foregoing; monoglycerides; diglycerides; triglycerides; sphingolipids; terpenes; steroids; and prostaglandins.

The composition for external application according to the present embodiment has the fibroblast-activating effect, the reducing effect of the oxidative stress induced cell damage, and the tyrosinase-inhibiting effect; therefore, it is preferably for dermal use. Examples of the dermal composition for external application include a dermal agent for external application and a dermal cosmetic.

The composition for external application according to the present embodiment may be in any form of solid, liquid (solution or suspension), and paste,. For example, it can be provided in a variety of dosage forms such as a liquid preparation, a suspension, an emulsion, an ointment, and a plaster. Further, when the composition for external application according to the present embodiment is a cosmetic, it can be made into a dosage form such as a cream, a lotion, a milky lotion, an ointment, a serum, a toner, a gel, a pack, or a foundation.

The composition for external application according to the present embodiment can be produced by compounding at least one component selected from the group consisting of trehalulose, isomaltulose, and reduced isomaltulose and other ingredient mentioned above as necessary.

The composition for external application according to the present embodiment has the fibroblast-activating effect, the reducing effect of the oxidative stress induced cell damage, and the tyrosinase-inhibiting effect; therefore, it can be used as an agent for activating fibroblast, an agent for reducing the oxidative stress induced cell damage, or an agent for inhibiting tyrosinase.

The agent for activating fibroblast, the agent for reducing the oxidative stress induced cell damage, and the agent for inhibiting tyrosinase according to the present embodiment may also be an agent which comprises, as an effective ingredient, at least one component selected from the group consisting of trehalulose, isomaltulose, and reduced isomaltulose. In the present embodiment, the agent for activating fibroblast, the agent for reducing the oxidative stress induced cell damage, and the agent for inhibiting tyrosinase may only consist of one component selected from the group consisting of trehalulose, isomaltulose, and reduced isomaltulose. Alternatively, it may further comprise the other ingredient mentioned above. Since trehalulose, isomaltulose, and reduced isomaltulose exert the above-described effects, they are useful as raw materials of cosmetics.

According to the present invention the composition is for use in a method of activating fibroblasts, a method of reducing the oxidative stress induced cell damage, a method of inhibiting tyrosinase, a method of improving wrinkles or sagging, a method of preventing the formation of skin spots or freckles, a method of preventing aging of the skin resulting from the cell damage, or a method of skin whitening, each of which involves delivering to at least a part of the skin or the nail, the composition for external application comprising as an effective ingredient, at least one component selected from the group consisting of trehalulose, isomaltulose, and reduced isomaltulose.

The delivery of the composition for external application can be carried out by applying, spraying, or pasting it to at least a part of the skin or the nail. The subject to which the delivery is made may be at least a part of the skin, including the scalp, or the nail and is preferably the at least part of the skin, including the scalp.

The delivery of the composition for external application may be carried out at least once daily (e.g., once, twice, three times, four times, or five times) and is preferably carried out once or twice daily. The amount of the composition for external application to be used may be set appropriately, depending on the conditions of the skin or the nail as the subject to be, the age,and the sex,. For example, it may be from 0.04 mg to 20 g, preferably from 0.1 mg to 10 g, more preferably from 0.3 mg to 5 mg, and further preferably from 0.5 mg to 3 g per day, in terms of the amount of the effective ingredient per day.

Moreover, described herein is the use of at least one component selected from the group consisting of trehalulose, isomaltulose, and reduced isomaltulose in the manufacture of the agent for activating fibroblast, the agent for reducing the oxidative stress induced cell damage, and the agent for inhibiting tyrosinase, the improver of wrinkles or sagging, the agent for preventing the formation of skin spots or freckles, the agent for preventing aging of the skin resulting from the cell damage, or the skin-whitening agent, each of which consists of the composition for external application.

The specific embodiments of each of the above-described uses are as explained in connection with the composition for external application.

### Examples

The present invention will be more specifically described based on the examples hereinbelow.

In the examples that follow, the trehalulose, isomaltulose, and reduced isomaltulose were used as being described below.
Trehalulose: Palatinose molasses were separated by a two-stage ion exchange chromatography using a strong acidic cation exchange resin of the calcium type (Proceedings of the Research Society of Japan Sugar Refineries' Technologists, No. 36, 1988, p.87-94)
Isomaltulose: Crystalline palatinose (trade name - "Crystalline Palatinose IC" manufactured by Mitsui Sugar Co., Ltd.) was dissolved and recrystallized twice.
Reduced isomaltulose: Trade name - "Palatinit PN" manufactured by Mitsui Sugar Co., Ltd.

### [Example 1: Evaluation of Fibroblast-Activating Effect]

Normal human epidermal fibroblasts suspended in a DMEM medium containing a 5% calf serum (FBS) were inoculated in a 96-well plate at a density of 2.0 x 10⁴ cells per well. After 24 hours of the inoculation, the medium was exchanged with a 1% calf serum (FBS) -containing DMEM medium (each test solution) which contained trehalulose, isomaltulose, or reduced isomaltulose at the concentrations shown in Table 1 (i.e., each six types of concentrations including 0.000 w/v %). Note that the concentration of the each test solution was a concentration which does not display toxicity toward the normal human epidermal fibroblasts. Also, 5% FBS-containing DMEM media were used as positive controls (P.C.). After culturing for 48 hours, the medium was exchanged with a 1% FBS-containing DMEM medium which contained 0.4 mg/mL of 3-(4,5-dimethylthiazol-2-yl)-2,5-diphenyltetrazolium bromide (MTT) and cultured for 2 hours. The medium was removed, and when 2-propanol was added, blue formazan generated was extracted. Absorbance of the extract at 550 nm was measured to obtain the amount of blue formazan. The fibroblast-activating effects are shown in terms of relative values [(Index(%)] which were obtained when the amounts of blue formazan generated by the non-treated cells (i.e., each test solution with a concentration of trehalulose, isomaltulose, or reduced isomaltulose being 0.000 w/v%) were taken as 100. The significance test was carried out by Student's t-test.

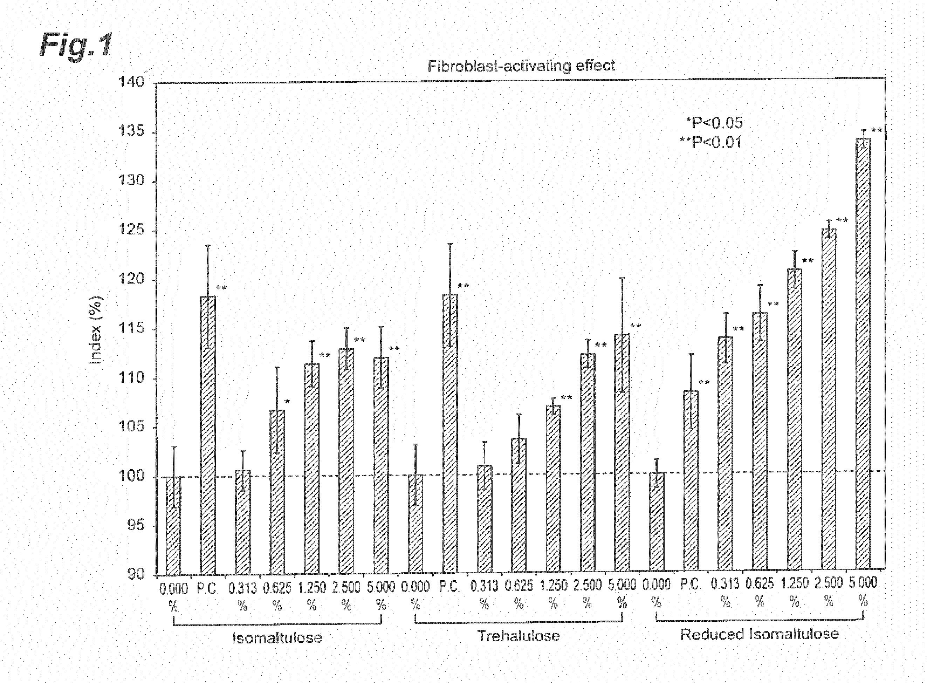

Results are shown in Table 1 as well as in FIG. 1. Each of trehalulose, isomaltulose, and reduced isomaltulose displayed significant fibroblast-activating effect. The fibroblast-activating effects were found to be concentration-dependent, and particularly, the concentration dependence was marked in reduced isomaltulose.

### [Table 1]

**Table 1. Fibroblast-activating effect (n=4)**

| Evaluation sample | Concentration (w/v%) | Index (%) | p Value |
|---|---|---|---|
| Trehalulose | 0.000 | 100.0 ± 3.1 | 1.000 |
| | P.C. | 118.3 ± 5.2 | 0.001 |
| | 0.313 | 100.9 ± 2.4 | 0.676 |
| | 0.625 | 103.6 ± 2.5 | 0.121 |
| | 1.250 | 106.9 ± 0.8 | 0.005 |
| | 2.500 | 112.2 ± 1.4 | 0.000 |
| | 5.000 | 114.1 ± 5.8 | 0.005 |
| Isomaltulose | 0.000 | 100.0 ± 3.1 | 1.000 |
| | P.C. | 118.3 ± 5.2 | 0.001 |
| | 0.313 | 100.6 ± 2.0 | 0.767 |
| | 0.625 | 106.7 ± 4.4 | 0.047 |
| | 1.250 | 111.4 ± 2.3 | 0.001 |
| | 2.500 | 112.9 ± 2.1 | 0.000 |
| | 5.000 | 112..0 ± 3.1 | 0.002 |
| Reduced isomaltulose | 0.000 | 100.0 ± 1.4 | 1.000 |
| | P.C. | 108.3 ± 3.8 | 0.006 |
| | 0.313 | 113.7 ± 2.5 | 0.000 |
| | 0.625 | 116.2 ± 2.8 | 0.000 |
| | 1.250 | 120.6 ± 1.9 | 0.000 |
| | 2.500 | 124.7 ± 0.9 | 0.000 |
| | 5.000 | 133.8 ± 0.9 | 0.000 |

### [Example 2: Evaluation of Reducing Effect of Oxidative Stress Induced Cell Damage]

When cells are irradiated with UVB, reactive oxygen species (such as a superoxide anion radical, hydrogen peroxide, and a hydroxyl radical) generate. The viability of cells obtained when the cells were irradiated with UVB was used as index to evaluate the reducing effect of the oxidative stress induced cell damage.

Normal human epidermal fibroblasts suspended in a KG2 medium were inoculated in a 96-well plate at a density of 2.0 x 10⁴ cells per well. After 24 hours of the inoculation, the medium was exchanged with a KG2 medium (each test solution) which contained trehalulose or reduced isomaltulose at the concentrations shown in Table 2 (i.e., each six types of concentrations including 0.000 w/v %) and cultured for 24 hours. Note that the concentration of the each test solution was a concentration which does not display toxicity toward the normal human epidermal cells. After culturing, the medium was exchanged with a Hanks' buffer which did not contain either Ca²⁺ or Mg²⁺, and after irradiation with UVB at 20mJ/cm², the medium was exchanged with the each test solution again and was cultured for additional 24 hours. Also, groups that were not irradiated with UVB were provided as control groups. After culturing, the medium was exchanged with a KB2 medium containing 33 µg/mL of Neutral Red (NR) and was cultured for 2 hours. After culturing, the cells were washed with PBS, and NR captured within the cells was extracted with a 0.1M HCl solution containing 30% (v/v) ethanol. Absorbance of the extract at 550 nm was measured. The reducing effect of cell damage is shown in terms of relative values [(Index (%)] which were obtained when the absorbance of the UVB non-irradiated group at each concentration was taken as 100. The significance test was carried out by Student's t-test.

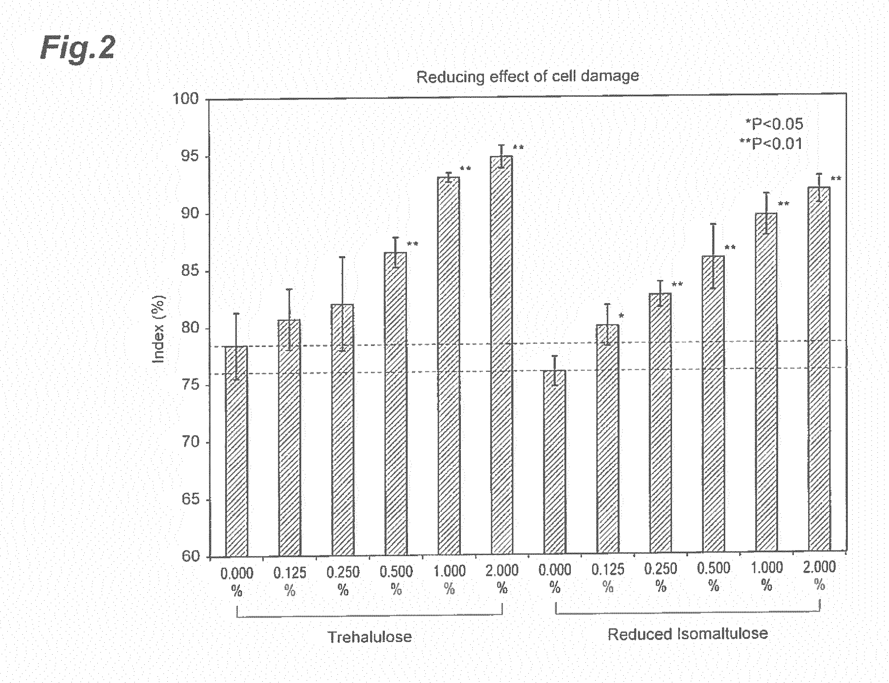

Results are shown in Table 2 as well as in FIG. 2. Either of trehalulose and reduced isomaltulose displayed significant reducing effect of cell damage. Said reducing effect of cell damage was found to be markedly concentration-dependent.

### [Table 2]

**Table 2. Reducing effect of oxidative stress induced cell damage (n=4)**

| Evaluation sample | Concentration (w/v%) | Index (%) | p Value |
|---|---|---|---|
| Trehalulose | 0.000 | 78.4 ± 2.9 | 1.000 |
| | 0.125 | 80.7 ± 2.7 | 0.300 |
| | 0.250 | 82.0 ± 4.1 | 0.200 |
| | 0.500 | 86.5 ± 1.3 | 0.002 |
| | 1.000 | 93.0 ± 0.4 | 0.000 |
| | 2.000 | 94.8 ± 1.0 | 0.000 |
| Reduced isomaltulose | 0.000 | 76.0 ± 1.3 | 1.000 |
| | 0.125 | 80.0 ± 1.8 | 0.011 |
| | 0.250 | 82.7 ± 1.1 | 0.000 |
| | 0.500 | 85.9 ± 2.8 | 0.001 |
| | 1.000 | 89.6 ± 1.8 | 0.000 |
| | 2.000 | 91.8 ± 1.2 | 0.000 |

### [Example 3: Evaluation of Tyrosinase-Inhibiting Effect]

Normal human melanocytes were dissolved in a phosphate buffer (100 mM, pH 6.8) containing 0.5% (w/v) Triton X-100 at a concentration of 6.0 x 10⁵ cells/mL, which was made a crude enzyme solution of tyrosinase. The DOPA oxidase activity of the crude enzyme solution was measured to evaluate the effect of a test sample that influences the enzyme activity of tyrosinase. Also, 5 mM kojic acid was used as positive controls (P.C.).

Each 50 µl of the crude enzyme solution was dispensed into a 96-well plate, and reaction was initiated by adding thereto, 0.5 mM L-DOPA [which was dissolved in a phosphate buffer (100 mM, pH 6.8)] and trehalulose or isomaltulose so that the respective concentrations shown in Table 3 were attained. Immediately after the initiation of reaction and after incubation at 37 °C for 2 hours, absorbances at 550 nm were measured with a microplate reader. This difference was used to calculate the amount of melanin based on the calibration curve prepared from the commercial synthetic melanin. The DOPA oxidase activity is shown as the amount of melanin formed per unit time and per unit protein mass of the crude enzyme solution: the tyrosinase enzyme activity is equal to µg of melanin/µg of protein/h. The significance test was carried out by Student's t-test.

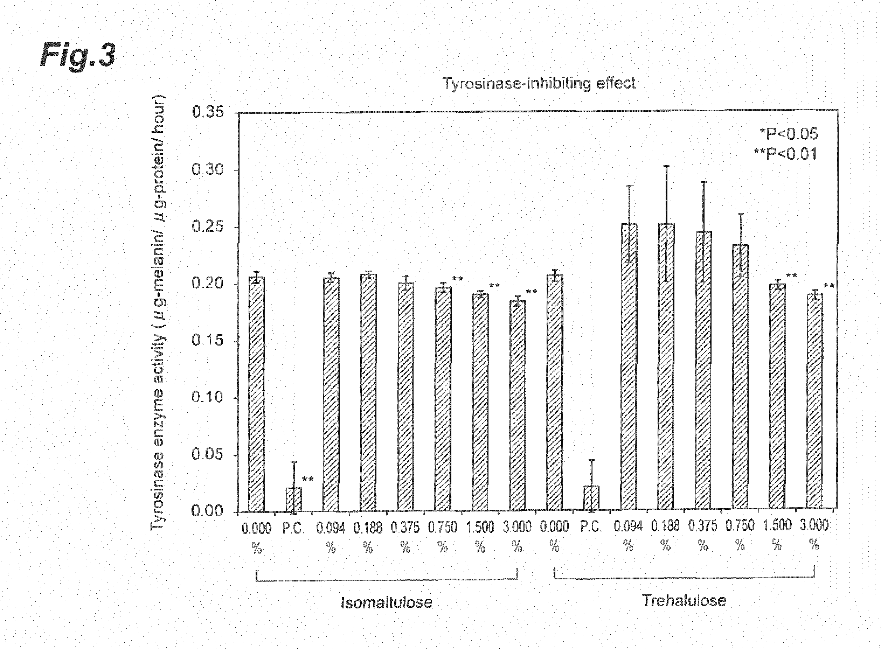

Results are shown in Table 3 as well as in FIG. 3. Either of trehalulose and isomaltulose displayed significant tyrosinase-inhibiting effect.

### [Table 3]

**Table 3. Tyrosinase-inhibiting effect (n=5)**

| Evaluation sample | Concentration (w/v%) | Tyrosinase enzyme activity (µg melanin/µg protein/h) | p Value |
|---|---|---|---|
| Trehalulose | 0.000 | 0.206 ± 0.005 | 1.000 |
| | Positive Control | 0.021 ± 0.023 | 0.000 |
| | 0.094 | 0.251 ± 0.034 | 0.020 |
| | 0.188 | 0.251 ± 0.051 | 0.085 |
| | 0.375 | 0.244 ± 0.044 | 0.090 |
| | 0.750 | 0.232 ± 0.028 | 0.075 |
| | 1.500 | 0.197 ± 0.004 | 0.010 |
| | 3.000 | 0.188 ± 0.004 | 0.000 |
| Isomaltulose | 0.000 | 0.206 ± 0.005 | 1.000 |
| | Positive Control | 0.021 ± 0.023 | 0.000 |
| | 0.094 | 0.205 ± 0.004 | 0.768 |
| | 0.188 | 0.208 ± 0.003 | 0.536 |
| | 0.375 | 0.200 ± 0.006 | 0.087 |
| | 0.750 | 0.196 ± 0.004 | 0.006 |
| | 1.500 | 0.190 ± 0.003 | 0.000 |
| | 3.000 | 0.184 ± 0.004 | 0.000 |

### [Example 4: Evaluation of Reducing Effect of Oxidative Stress Induced Cell Damage]

Except that the each test solution was changed to a KG2 medium which contained isomaltulose at the concentrations shown in Table 4 (i.e., six types of concentrations including 0.000 w/v %) and that the irradiation intensity of UVB was changed to 25 mJ/cm², the reducing effect of the oxidative stress induced cell damage was evaluated according to the same procedure as in Example 2.

### [Table 4]

**Table 4. Reducing effect of oxidative stress induced cell damage (n=4)**

| Evaluation sample | Concentration (w/v%) | Index (%) | p Value |
|---|---|---|---|
| Isomaltulose | 0.000 | 76.8 ± 4.6 | 1.000 |
| | 0.013 | 80.0 ± 1.8 | 0.242 |
| | 0.025 | 83.7 ± 3.5 | 0.054 |
| | 0.050 | 84.3 ± 1.1 | 0.020 |
| | 0.100 | 86.2 ± 2.5 | 0.012 |
| | 0.200 | 86.8 ± 2.9 | 0.011 |

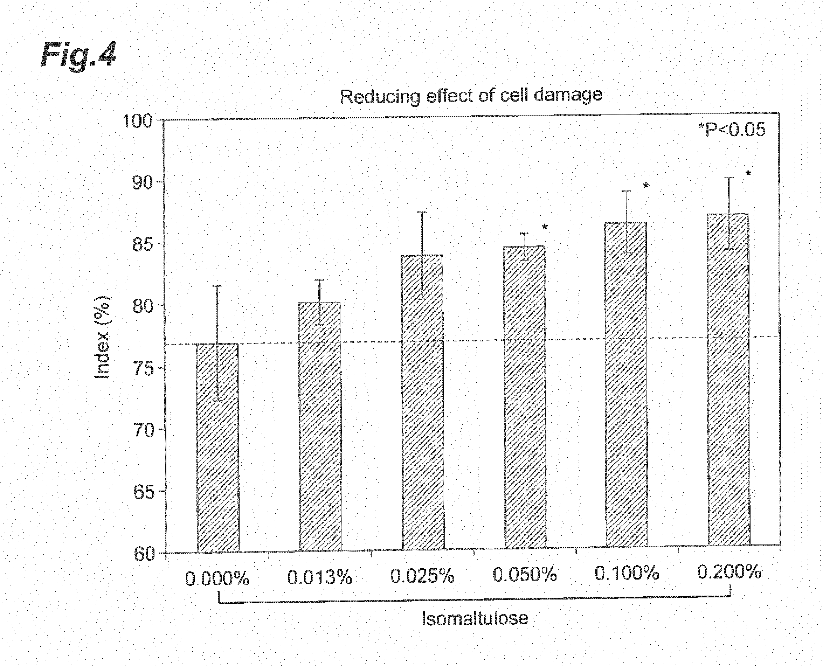

Results are shown in Table 4 as well as in FIG. 4. Isomaltulose also displayed significant reducing effect of cell damage. Said reducing effect of cell damage was found to be markedly concentration-dependent.

## Claims

1. Cosmetic use of a composition for external application which comprises as an effective ingredient at least one component selected from the group consisting of trehalulose and isomaltulose, for improving wrinkles or sagging.

2. Composition for external application which comprises as an effective ingredient at least one component selected from the group consisting of trehalulose, isomaltulose, and reduced isomaltulose for use in a method for reducing cell damage induced by oxidative stress caused by ultraviolet rays.

3. Cosmetic use of a composition for external application which comprises as an effective ingredient at least one component selected from the group consisting of trehalulose, isomaltulose, and reduced isomaltulose for preventing the formation of skin Z spots or freckles.

4. Cosmetic use of a composition for external application which comprises as an effective ingredient at least one component selected from the group consisting of trehalulose, isomaltulose and reduced isomaltulose for whitening the skin.

## Patentansprüche

1. Kosmetische Verwendung einer Zusammensetzung zur äußerlichen Anwendung, die als wirksamen Bestandteil mindestens eine Komponente, ausgewählt aus der Gruppe bestehend aus Trehalulose und Isomaltulose, enthält, zur Verbesserung von Falten oder erschlaffender Haut.

2. Zusammensetzung zur äußerlichen Anwendung, die als wirksamen Bestandteil mindestens eine Komponente, ausgewählt aus der Gruppe bestehend aus Trehalulose, Isomaltulose und reduzierter Isomaltulose, enthält, zur Verwendung in einem Verfahren zur Verringerung von Zellschäden, die durch oxidativen Streß, verursacht durch ultraviolette Strahlen, induziert werden.

3. Kosmetische Verwendung einer Zusammensetzung zur äußerlichen Anwendung, die als wirksamen Bestandteil mindestens eine Komponente, ausgewählt aus der Gruppe bestehend aus Trehalulose, Isomaltulose und reduzierter Isomaltulose, enthält, um die Bildung von Flecken oder Sommersprossen zu verhindern.

4. Kosmetische Verwendung einer Zusammensetzung zur äußerlichen Anwendung, die als wirksamen Bestandteil mindestens eine Komponente, ausgewählt aus der Gruppe bestehend aus Trehalulose, Isomaltulose und reduzierter Isomaltulose, enthält, zur Aufhellung der Haut.

## Revendications

1. Utilisation cosmétique d'une composition pour application externe qui comprend comme ingrédient efficace au moins un composant sélectionné dans le groupe constitué du tréhalulose et de l'isomaltulose, pour améliorer les rides ou l'affaissement.

2. Composition pour application externe qui comprend comme ingrédient efficace au moins un composant sélectionné dans le groupe constitué du tréhalulose, de l'isomaltulose, et de l'isomaltulose réduit pour l'utilisation dans un procédé de réduction des dégâts cellulaires induits par le stress oxydatif provoqué par les rayons ultraviolets.

3. Utilisation cosmétique d'une composition pour application externe qui comprend comme ingrédient efficace au moins un composant sélectionné dans le groupe constitué du tréhalulose, de l'isomaltulose, et de l'isomaltulose réduit pour empêcher la formation des taches cutanées ou des taches de rousseur.

4. Utilisation cosmétique d'une composition pour application externe qui comprend comme ingrédient efficace au moins un composant sélectionné dans le groupe constitué du tréhalulose, de l'isomaltulose, et de l'isomaltulose réduit pour le blanchissement de la peau.
